Europäisches Patentamt

(19) European Patent Office    (11) Publication number:    **0 149 508**

Office européen des brevets    **B1**

(12)    **EUROPEAN PATENT SPECIFICATION**

(45) Date of publication of patent specification: **23.03.88**    (51) Int. Cl.⁴: **C 07 C 6/12,** C 07 C 15/02, C 07 C 39/08, C 07 C 37/08 // C01B33/28

(21) Application number: **85300013.1**

(22) Date of filing: **02.01.85**

(54) **Production of para-diisopropylbenzene and use of the same in the production of hydroquinone.**

(30) Priority: **04.01.84 US 568010**
**04.01.84 US 568009**

(43) Date of publication of application:
**24.07.85 Bulletin 85/30**

(45) Publication of the grant of the patent:
**23.03.88 Bulletin 88/12**

(84) Designated Contracting States:
**BE DE FR GB IT NL**

(56) References cited:
**GB-A- 764 338**
**US-A-2 748 172**
**US-A-3 832 449**
**US-A-4 329 509**
**US-A-4 367 359**
**US-A-4 380 658**

The file contains technical information
submitted after the application was filed and
not included in this specification

(73) Proprietor: **MOBIL OIL CORPORATION**
**150 East 42nd Street**
**New York New York 10017 (US)**

(72) Inventor: **Kaeding, Warren William**
**6 Roseberry Court**
**Lawrenceville New Jersey 08648 (US)**

(74) Representative: **Cooper, John Anthony et al**
**Mobil Court 3 Clements Inn**
**London WC2A 2EB (GB)**

Courier Press, Leamington Spa, England.

## Description

This invention relates to a process for the selective production of para-diisopropylbenzene by catalytic disproportionation of cumene and the conversion of the resultant para-diisopropylbenzene to hydroquinone.

Both para-diisopropylbenzene and hydroquinone have a number of commercial uses; para-diisopropylbenzene as a solvent and chemical intermediate and hydroquinone in photographic developers, medicines, dye intermediates, anti-oxidants, inhibiters, and stabilizers.

The disproportionation of aromatic hydrocarbons in the presence of zeolite catalysts has been described by Grandio et al. in the *Oil and Gas Journal,* Vol. 69, Number 48 (1971). In addition, U.S. Pat. Nos. 3,126,422; 3,413,374; 3,589,878; 3,589,879; and 3,607,961 show vapor phase disproportionation of toluene over various catalysts.

U.S. Pat. No. 4,011,276 disclosed disproportionation of toluene to produce benzene and xylenes rich in the para isomer with a catalyst comprising of crystalline aluminosilicate zeolite of the ZSM-5 type which; has been modified by the addition thereto of a minor proportion of an oxide of magnesium. U.S. Patent No. 4,016,219 discloses a similar reaction but wherein the catalyst employed has been modified by the addition thereto of phosphorus in an amount of at least about 0.5 percent by weight. While the process of each of these patents show selective production of para-xylene, this is obtained only by tolerating a very substantial reduction in toluene conversion, i.e., decrease in activity compared to disproportionation of toluene carried out under comparable conditions with the unmodified zeolite catalyst.

In these prior art processes, the xylene product produced either has the equilibrium composition of approximately 24 percent of para, 54 percent of meta and 22 percent of ortho or, in those instances where the para isomer is produced in an amount in excess of its equilibrium concentration, such is achieved only at great expense of activity, i.e., a very substantial reduction in toluene conversion. Of the xylene isomers, i.e., ortho-, meta- and para-xylene, meta xylene is the least desired product, with ortho- and para-xylene being the more desired products. Para-xylene, in substantial yield, is of particular value being useful in the manufacture of terephthalic acid which is an intermediate in the manufacture of synthetic fibers such as "Dacron". Mixtures of xylene isomers either alone or in further admixture with ethylbenzene have previously been separated by expensive superfractionation and multistage refrigeration steps. Such process, as will be realized, has involved high operation costs and has a limited yield.

It is also known from US Patent 4,367,359 that zeolites having a constraint index of 1—12 can be used in the selective conversion of a wide variety of aromatic feeds, including propylbenzene, to para-dialkylbenzenes. One of the zeolites disclosed in this reference as having a constraint index within the required range of 1—12 is ZSM-12, but the preferred zeolite is ZSM-5.

Moreover, it is known from US Patent No. 2,748,172 that oxidation of para-diisopropylbenzene produces the corresponding dihydroperoxide which then can undergo rearrangement to produce hydroquinone and acetone.

In accordance with one aspect of the present invention, there is provided a process for the selective disproportionation of cumene with the selective production of para-diisopropylbenzene, said process comprising contacting said cumene with ZSM-12 catalyst under disproportionation conditions.

Suitable disproportionation conditions may include a temperature between 390°F (200°C) and 1,400°F (760°C) at a pressure between atmospheric and 1,000 psig (6,996 rPa) utilizing a feed weight hourly space velocity (WHSV) between 0.08 and 2.0. The latter WHSV is based upon the weight of catalyst composition, i.e., total weight of active catalyst and binder therefor. The effluent is separated and distilled to remove undesired products and to separate benzene, cumene and diisopropylbenzene. Unreacted cumene may be recycled.

In a further aspect, the invention resides in a process for the selective production of hydroquinone, said process comprising the steps of:

(i) contacting cumene with a ZSM-12 catalyst under disproportionating conditions to selectively produce para-diisopropylbenzene and benzene;

(ii) oxidizing said para-diisopropylbenzene of step (i) to the corresponding dihydroperoxide; and

(iii) rearranging said dihydroperoxide product of step (ii) to produce hydroquinone and acetone.

The crystalline zeolite utilized herein is ZSM-12, the characteristics and synthesis of which are described in, for example, U.S. Patent No. 3,832,449, and EP—B—18089.

When prepared in the presence of organic cations, ZSM-12 is substantially catalytically inactive, possibly because the intracrystalline free space is occupied by organic cations from the forming solution. It may be activated by heating in an inert atmosphere at 540°C for one hour, for example, followed by base exchange with ammonium salts followed by calcination at 540°C in air. The presence of organic cations in the forming solution may not be absolutely essential to the formation of the zeolite. More generally, it is desirable to activate this type of catalyst by base exchange with ammonium salts followed by calcination in air at about 549°C for from 15 minutes to 24 hours.

When synthesized in the alkali metal form, the zeolite is conveniently converted to the hydrogen form, generally by intermediate formation of the ammonium form as a result of ammonium ion exchange and calcination of the ammonium form to yield the hydrogen form. In addition to the hydrogen form, other forms of the zeolite wherein the original alkali metal has been reduced to less than about 1.5 per cent by weight may be used. Thus, the original alkali metal of the zeolite may be replaced by ion exchange with other suitable ions of Groups IB to VIII of the Periodic Table, including by way of example, nickel, copper, zinc, palladium, calcium or rare earth metals.

In practicing the present process, it may be desirable to incorporate the above described crystalline zeolite in another material resistant to the temperature and other conditions employed in the process. Such matrix materials include synthetic or naturally occurring substances as well as inorganic materials such as clay, silica and/or metal oxides. The latter may be either naturally occurring or in the form of gelatinous precipitates or gels including mixtures of silica and metal oxides. Naturally occurring clays which can be composited with the zeolite include those of the montmorillonite and kaoline families, which families include the sub-bentonites and the kaolins commonly known as Dixie, McNamee-Georgia and Flordia clays or others in which the main mineral constituent is halloysite, kaolinite, dickite, nacrite or anauxite. Such clays can be used in the raw state as originally mined or initially subjected to calcination, acid treatment or chemical modification.

In addition to the foregoing materials, the zeolite employed herein may be composited with a porous matrix material, such as alumina, silica-alumina, silica-magnesia, silica-zirconia, silica-thoria, silica-beryllia, silica-titania as well as ternary compositions, such as silica-alumina-thoria, silica-alumina-zirconia, silica-alumina-magnesia and silica-magnesia-zirconia. The matrix may be in the form of a cogel. The relative proportions of zeolite component and inorganic oxide gel matrix on an anhydrous basis may vary widely with the zeolite content ranging from between 1 to 99 percent by weight and more usually in the range of 5 to 80 percent by weight of the dry composite.

A second optional component of the ZSM-12 catalyst comprises a minor proportion, e.g., from 0.05% to 50% by weight of the catalyst composite, of a difficultly reducible oxide. Oxides of this type can include oxides of phosphorus as well as those oxides of the metals of Groups IA, IIA, IIIA, IVA, VA, VIA, VIIA, VIIIA, IB, IIB, IIIB, IVB, or VB of the Periodic Chart of the Elements (Fisher Scientic Company, Catalog No. 5-702-10) which serve to enhance the para-selectivity properties of the catalysts modified therewith. The difficulty reducible oxides most commonly employed to modify the selectivity properties of the catalyst are oxides of phosphorus and magnesium. Thus, the catalyst can be treated with phosphorus and/or magnesium compounds in the manner described in U.S. Patent Nos. 3,894,104; 4,049,573; 4,086,287; and 4,128,592.

Phosphorus, for example, can be incorporated into the catalyst at least in part in the form of phosphorus oxide in an amount of from 0.25% to 25% by weight of the catalyst composition, preferably from 0.7% to 15% by weight. Such incorporation can be readily effected by contacting the zeolite composite with a solution of an appropriate phosphorus compound, followed by drying and calcining to convert phosphorus compound, followed by drying and calcining to convert phosphorus in the zeolite to its oxide form. Preferred phosphorus-containing compounds include diphenyl phosphine chloride, trimethylphosphite and phosphorus trichloride', phosphoric acid, phenyl phosphine oxychloride, trimethylphosphate, diphenyl phosphinous acid, diphenyl phosphinic acid, diethylchlorothinophosphate, methyl acid phosphate and other alcohol-$P_2O_5$ reaction products. Particularly preferred are ammonium phosphates, including ammonium hydrogen phosphate, $(NH_4)_2HPO_4$, and ammonium dihydrogen phosphate, $NH_4H_2PO_4$. Calcination is generally conducted in the presence of oxygen at a temperature of at least about 150°C. However, higher temperatures, i.e., up to 500°C or higher are preferred. Such heating is generally carried out for 3—5 hours but may be extended to 24 hours or longer.

Magnesium oxide is another preferred difficulty reducible oxide which can be incorporated with the zeolite composite in a manner similar to that employed with phosphorus. Magnesium can comprise from 0.25% to 25% by weight preferably from 1% to 15% by weight present at least in part as magnesium oxide. As with phosphorus, magnesium oxide incorporation is effected by contacting the zeolite composite with an appropriate magnesium compound followed by drying and calcining to convert magnesium in the zeolite to its oxide form. Preferred magnesium-containing compounds include magnesium nitrate and magnesium acetate. Calcination times and temperatures are generally the same as recited hereinbefore for calcination of the phosphorus-containing catalyst.

In addition to treatment of the zeolite to incorporate phosphorus and/or magnesium oxides, the zeolite may also be modified in a substantially similar manner to incorporate thereon a variety of other oxide materials to enhance para-selectivity. Such oxide materials include oxides of boron (U.S. 4,067,920); antimony (U.S. 3,979,472); beryllium (U.S. 4,260,843); Group VIIA Metals (U.S. 4,275,256); alkaline earth metals (U.S. 4,288,647); Group IB metals (U.S. 4,276,438); Group IVB metals (U.S. 4,278,827); Group VIA metals (U.S. 4,259,537); Group IA elements (U.S. 4,329,533); cadmium (U.S. 4,384,155); Group IIIB metals (U.S. 4,276,437); Group IVA metals (U.S. 4,302,620); Group VA metals (U.S. 4,302,621); and Group IIIA elements (U.S. 4,302,622).

By means of the present disproportionation reaction, it is possible to obtain a diisopropylbenzene product having, e.g., at least 60 percent of the para-isomer and, e.g., less than 1 percent of the ortho-osomer.

The process may be conducted with the cumene reactant in either the gaseous or the liquid phase. It may be carried out as a batch-type, semi-continuous or continuous operation utilizing a fixed, fluidized or moving bed catalyst system. The Example which follows will serve to illustrate the process of the invention.

## Example

. A steamed HZSM-12 catalyst having a silica to alumina ratio of 180, was tested for disproportionation of cumene. The hydrogen form of the zeolite was prepared by calcination, $NH_4$+ion exchange and final calcination. The zeolite was steamed for 3 hours at 1000°F (538°C), 100 percent steam and atmospheric pressure. The ZSM-12 was intimately mixed with 35 wt.% alumina binder, then pressed into wafers, crushed and screened to a uniform particle size of 14—20 mesh.

Reaction conditions and results are summarized in Table 1, wherein "CU" stands for cumene, "DIPB" stands for diisopropylbenzene and "NPRBZ" stands for n-propylbenzene.

TABLE 1
Cumene Disproportionation

| Run No. | 1 | 2 | 3 | 4 |
|---|---|---|---|---|
| Temp., °C | 150 | 150 | 200 | 250 |
| Pressure, pisg (kPa) | 500(3549) | 500(3549) | 500(3549) | 500(3549) |
| CU WHSV | 3.4 | 3.4 | 3.4 | 3.4 |
| Conversion, wt.% CU | 3.2 | 2.6 | 13.2 | 45.1 |
| Selectivity, wt% Benzene | 33.3 | 39.7 | 33.6 | 34.2 |
| DIPB para | 14.7 | 14.1 | 26.9 | 21.5 |
| meta | 9.6 | 9.2 | 31.6 | 38.9 |
| ortho | 0 | 0 | .7 | .5 |
| Total | 24.3 | 23.3 | 59.2 | 60.9 |
| NORBZ | 4.9 | 3.4 | .7 | .7 |
| Other* | 37.5 | 33.6 | 6.5 | 4.2 |
| DIPB % para | 60.5 | 60.6 | 45.5 | 35.3 |
| meta | 39.5 | 39.4 | 53.4 | 63.9 |
| ortho | 0 | 0 | 1.2 | .8 |

*Primarily propylene oligomers

Conversion was relatively low at 150°C (3%) but increased to 13 and 45% at 200 and 250°C. Approximately equimolar amounts of benzene and DIPB were produced. At the latter higher temperatures, significantly more meta isomer (53—64%), compared with par (46—35%), was observed. The co-product benzene could be converted to cumene by alkylation with propylene for recycle. This could be a particularly practical extra step if a cumene plant were conveniently located.

The para-diisopropylbenzene product of the foregoing Example may be oxidized, e.g., with air, to the corresponding dihydroperoxide and rearranged by an acid catalyst to hydroquinone and acetone. The meta isomer is converted to resorcinol and acetone by an analogous process.

## Claims

1. A process for the selective disproportionation of cumene with the selective production of para-diisopropylbenzene, said process comprising contacting said cumene with ZSM-12 catalyst under disproportionating conditions.

2. A process for the selective production of hydroquinone, said process comprising the steps of:

4

(i)   contacting cumene with a ZSM-12 catalyst under disproportionating conditions to selectively produce para-diisopropylbenzene and benzene;

(ii)  oxidizing said para-diisopropylbenzene of step (i) to the corresponding dihydroperoxide; and

(iii) rearranging said dihydroperoxide product of step (ii) to produce hydroquinone and acetone.

**Patentansprüche**

1. Verfahren zur selektiven Disproportionierung von Cumol mit selektiver Herstellung von p-Diisopropylbenzol, wobei das Verfahren den Kontakt von Cumol mit einem ZSM-12-Katalysator unter Disproporionierungsbedingungen umfaßt.

2. Verfahren zur selektiven Herstellung von Hydrochinon, wobei das Verfahren die Schritte umfaßt:

(i) Kontakt des Cumols mit einem ZSM-12-Katalysator unter Disproportionierungsbedingungen, um p-Diisopropylbenzol und Benzol selektiv herzustellen,

(ii) Oxidation des p-Diisopropylbenzols von Schritt (i) zum korrespondierenden Dehydroperoxid und

(iii) Umlagerung des Dihydroperoxidprodukts von Schritt (ii) um Hydrochinon und Azeton herzustellen.

**Revendications**

1. Un procédé de disproportionation sélective de cumène avec production sélective de para-diisopropylbenzène, ce procédé consistant à mettre le cumène au contact d'un catalyseur à base de ZSM-12 dans des conditions de disproportionation.

2. Un procédé de préparation sélectiv'e d'hydroquinone, ce procédé consistant à:

(i) mettre le cumène au contact d'un catalyseur à base de ZSM-12 dans les conditions de disproportionation pour préparer sélectivement du para-diisopropylbenzène et du benzène;

(ii) oxyder ce para-diisopropylbenzène provenant de l'étape (i) en le dihydroperoxyde correspondant; et

(iii) ré-arranger le dihydroperoxyde obtenu dans l'étape (ii) pour préparer l'hydroquinone et l'acétone.